# EUROPEAN PATENT APPLICATION

(11) **EP 4 799 578 A1**
(43) Date of publication of application: **02.09.2026**
(21) Application number: 24909997.9
(22) Date of filing: 10.09.2024
(51) Int. Cl.: A61B 17/32

(54) **CUTTING BALLOON AND MEDICAL DEVICE**

(30) Priority: 25.12.2023 CN 202311805275; 25.12.2023 CN 202323554745 U
(71) Applicant: Shanghai Shenqi Medical Technology Co., Ltd., Shanghai 201201 (CN)
(72) Inventor: ZHANG, Jinxia, Shanghai 201201 (CN); SONG, Rui, Shanghai 201201 (CN); WANG, Sen, Shanghai 201201 (CN); FAN, Jun, Shanghai 201201 (CN)
(74) Representative: J A Kemp LLP
(86) International application number: PCT/CN2024/117934
(87) International publication number: WO 2025/139051

(57) **Abstract**

Provided are a cutting balloon and a medical device. The cutting balloon includes a balloon body (1), a plurality of cutter assemblies (2), and a hydrophilic coating (4). The plurality of cutter assemblies (2) are arranged on an outer wall of the balloon body (1) and distributed at intervals along a circumferential direction of the balloon body (1). The hydrophilic coating (4) is disposed on the outer wall of the balloon body (1). When the cutting balloon moves within a blood vessel, in a wet state, the hydrophilic coating (4) is activated and exhibits excellent lubricating properties upon contact with blood, which can improve the crossing performance of the cutter assembly (2) of the cutting balloon in complex and tortuous positions of the blood vessel. Thus, the success rate of crossing is improved, thereby improving the safety and convenience of a surgical operation.

## Description

This application claims priority to Chinese Patent Application No. 202311805275.8 filed with the China National Intellectual Property Administration (CNIPA) on Dec. 25, 2023 and Chinese Patent Application No. 202323554745.6 filed with the CNIPA on Dec. 25, 2023, which are incorporated herein by reference in their entireties.

### TECHNICAL FIELD

The present application relates to the field of medical engineering technology and, in particular, to a cutting balloon and a medical device.

### BACKGROUND

Interventional surgeries have been widely used in clinical practice as an important means for treating cardiovascular diseases. When dilating a blood vessel, an ordinary balloon may cause geometric deformation and tearing of the intima and media of a stenotic segment of the blood vessel, resulting in compression and rupture of a plaque and elastic expansion of the blood vessel. This easily leads to intimal tearing or even acute occlusion. Subsequent elastic recoil and proliferative responses to injury may result in restenosis.

A cutting balloon is a special type of balloon that combines micro-incision technology with balloon dilation. When the balloon is inflated, a cutting assembly is pushed outward to cut a soft plaque in the blood vessel and the intima of the blood vessel to a certain extent. This facilitates balloon inflation and vessel dilation with less applied force, thereby avoiding vascular rupture caused by excessive balloon inflation. Compared with the dilation performed by the ordinary balloon, the cutting balloon can reduce an inflammatory response, elastic recoil, intimal injury of the blood vessel, proliferative responses of smooth muscle cells of the blood vessel, and a potential risk of restenosis. In addition, the cutting balloon has the function of gaining a larger lumen area of the blood vessel, with the potential to increase a technical success rate, improve vessel patency, and achieve better long-term outcomes.

However, in the related art, cutting balloons exhibit poor crossability within blood vessels and have difficulty crossing smoothly through tortuous positions, which may cause intimal injury of the blood vessels or product damage, affecting the safety and convenience of a surgical operation.

Based on this, there is an urgent need for a cutting balloon and a medical device to solve the existing problems described above.

### SUMMARY

Based on the preceding description, an object of the present application is to provide a cutting balloon and a medical device, which can improve the crossing performance of a cutter assembly of the cutting balloon in complex and tortuous positions of a blood vessel and improve the crossability of the cutting balloon.

To achieve the preceding object, the present application adopts the technical solutions described below.

On the one hand, a cutting balloon is provided.

The cutting balloon comprises a balloon body and a plurality of cutter assemblies.

The plurality of cutter assemblies are arranged on an outer wall of the balloon body and distributed at intervals along a circumferential direction of the balloon body.

A hydrophilic coating is disposed on the outer wall of the balloon body.

As an optional technical solution of the cutting balloon, the hydrophilic coating is disposed on outer walls of the plurality of cutter assemblies.

As an optional technical solution of the cutting balloon, a coupling agent is disposed between the hydrophilic coating and the outer wall of the balloon body, and one side of the coupling agent is connected to the outer wall of the balloon body and another side of the coupling agent is connected to the hydrophilic coating.

As an optional technical solution of the cutting balloon, the cutting balloon further comprises a first coating, wherein the first coating is disposed on the outer wall of the balloon body.

As an optional technical solution of the cutting balloon, the first coating is located between the balloon body and the plurality of cutter assemblies, and the plurality of cutter assemblies are bonded to the first coating.

As an optional technical solution of the cutting balloon, the first coating is disposed between the hydrophilic coating and the outer wall of the balloon body.

As an optional technical solution of the cutting balloon, a coupling agent is disposed between the hydrophilic coating and the first coating, and one side of the coupling agent is connected to the first coating and another side of the coupling agent is connected to the hydrophilic coating.

As an optional technical solution of the cutting balloon, the first coating is an acidic oxide.

As an optional technical solution of the cutting balloon, the coupling agent is a silane coupling agent, a titanate coupling agent, or an aluminate coupling agent.

As an optional technical solution of the cutting balloon, a material of the hydrophilic coating is polyacrylamide, polyvinylpyrrolidone, polyethylene glycol, polyvinyl alcohol, or natural polysaccharides.

As an optional technical solution of the cutting balloon, the first coating is capable of modifying a surface of the balloon body to increase surface roughness of the balloon body.

As an optional technical solution of the cutting balloon, roughness of the first coating ranges from Ra 0.8 to Ra 15.

As an optional technical solution of the cutting balloon, each cutter assembly among the plurality of cutter assemblies comprises a cutter holder and a blade, the blade is mounted on one side of the cutter holder, and the cutter holder is bonded to the first coating.

As an optional technical solution of the cutting balloon, each cutter assembly among the plurality of cutter assemblies comprises a cutter holder and a blade, the cutter holder extends along an axial direction of the balloon body, and two ends of the cutter holder are provided with U-shaped chamfers.

On the other hand, a medical device is provided. The medical device comprises the cutting balloon described in any one of the preceding solutions.

The present application has the beneficial effects described below.

The present application provides the cutting balloon and the medical device. When the cutting balloon moves within the blood vessel, in a wet state, the hydrophilic coating is activated and exhibits excellent lubricating properties upon contact with blood, which can improve the crossing performance of the cutter assembly of the cutting balloon in complex and tortuous positions of the blood vessel and improve the crossability of the cutting balloon. Thus, the success rate of crossing is improved during the advancement of the cutting balloon toward a lesion site or the withdrawal of the cutting balloon, thereby improving the safety and convenience of a surgical operation.

### BRIEF DESCRIPTION OF DRAWINGS

To illustrate technical solutions in embodiments of the present application more clearly, drawings used in the description of the embodiments of the present application are briefly described below. Apparently, the drawings described below illustrate only part of the embodiments of the present application, and those of ordinary skill in the art may obtain other drawings based on the content of the embodiments of the present application and the drawings on the premise that no creative work is done.
FIG. 1 is a structural view of a cutting balloon according to embodiment one of the present application.
FIG. 2 is a cross-sectional view of a cutting balloon according to embodiment one of the present application.
FIG. 3 is an enlarged view of part A in FIG. 2.
FIG. 4 is a partial structural cross-sectional view of a cutting balloon according to embodiment two of the present application.

The reference numerals in the drawings are as follows:
- 1: balloon body
- 2: cutter assembly
- 21: cutter holder
- 22: blade
- 3: first coating
- 4: hydrophilic coating

### DETAILED DESCRIPTION

The present application is further described below in detail in conjunction with the drawings and embodiments. It is to be understood that the embodiments described herein are intended to explain the present application and not to limit the present application. Additionally, it is to be noted that for convenience of description, only part, not all, of the structures related to the present application are illustrated in the drawings.

In the description of the present application, the terms "joined", "connected", and "fixed" are to be understood in a broad sense unless otherwise expressly specified and limited. For example, the term "connected" may refer to "fixedly connected", "detachably connected", or "integrated", may refer to "mechanically connected" or "electrically connected", or may refer to "connected directly", "connected indirectly through an intermediary", "connected inside two elements", or "an interaction relationship between two elements". For those of ordinary skill in the art, specific meanings of the preceding terms in the present application may be understood based on specific situations.

In the present application, unless otherwise expressly specified and limited, when a first feature is described as "on" or "under" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

In the description of this embodiment, orientations or position relations indicated by terms such as "on", "under", "left", and "right" are based on the drawings. These orientations or position relations are intended only to facilitate description and simplify operations and not to indicate or imply that a device or element referred to must have such specific orientations or must be configured or operated in such specific orientations. Thus, these orientations or position relations are not to be construed as limiting the present application. Additionally, the terms "first" and "second" are only used for distinguishing between descriptions and have no special meanings.

### Embodiment one

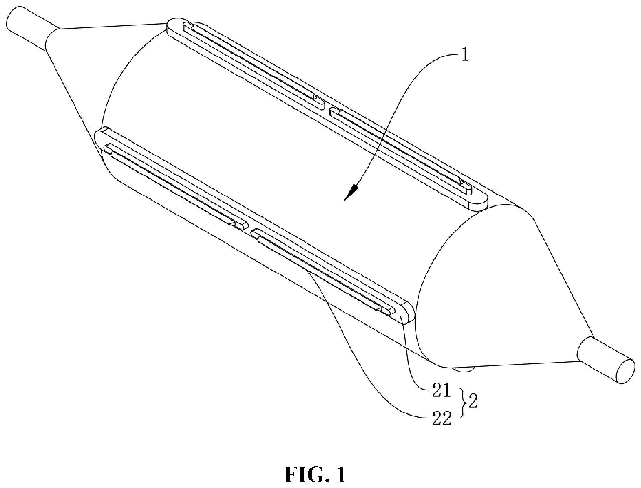

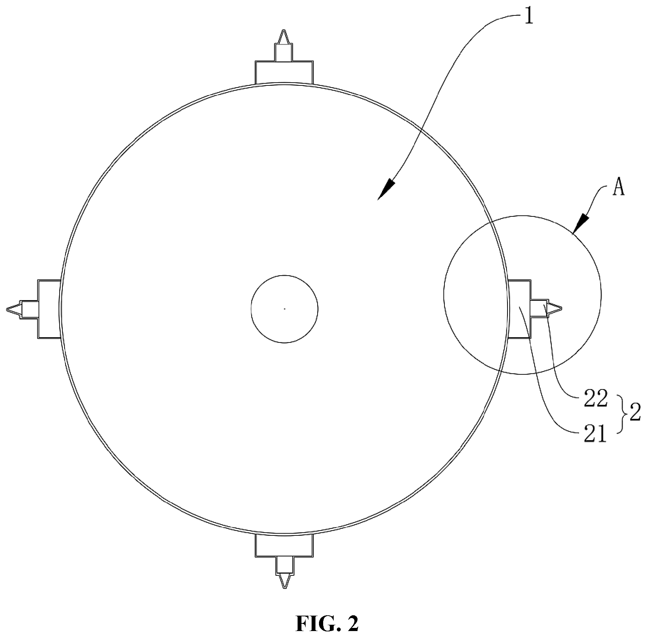

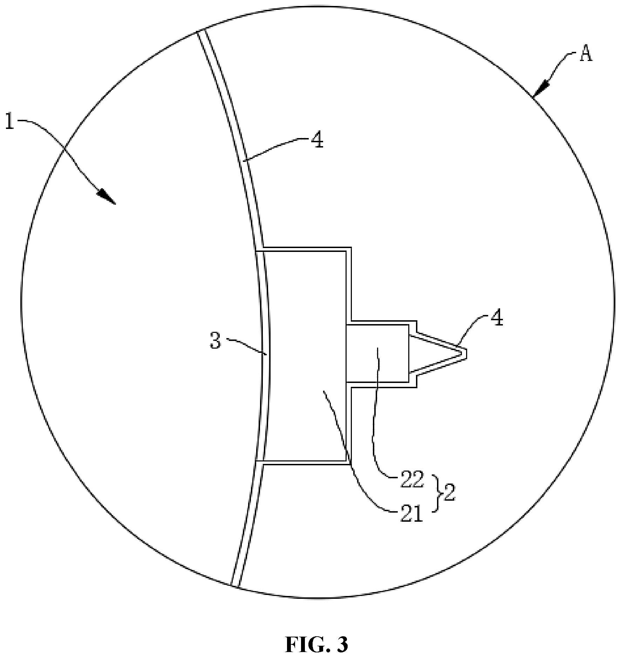

As shown in FIGS. 1 to 3, this embodiment provides a medical device including a cutting balloon. The cutting balloon comprises a balloon body 1, a cutter assembly 2, and a hydrophilic coating 4. A plurality of cutter assemblies 2 are arranged on the outer wall of the balloon body 1 and distributed at intervals along the circumferential direction of the balloon body 1. A hydrophilic coating 4 is disposed on the outer wall of the balloon body 1. When the cutting balloon moves within a blood vessel, in a wet state, the hydrophilic coating 4 is activated and exhibits excellent lubricating properties upon contact with blood, which can improve the crossing performance of the cutter assembly 2 of the cutting balloon in complex and tortuous positions of the blood vessel and improve the crossability of the cutting balloon. Thus, the success rate of crossing is improved during the advancement of the cutting balloon toward a lesion site or the withdrawal of the cutting balloon. Optionally, the hydrophilic coating 4 is disposed on the outer wall of the cutter assembly 2, thereby reducing the risk of vascular injury caused by the cutter assembly 2.

In this embodiment, a material of the hydrophilic coating 4 is polyacrylamide, polyvinylpyrrolidone, polyethylene glycol, polyvinyl alcohol, or natural polysaccharides and derivatives. The reason why the hydrophilic coating 4 improves lubricity is that the material of the hydrophilic coating 4 is a hydrophilic polymer. Molecules of the hydrophilic polymer contain hydrophilic groups, such as amide groups, ether linkages, and hydroxyl groups absorb water to form a hydration layer, thereby imparting hydrophilicity and lubricity to the hydrophilic coating 4.

Optionally, the hydrophilic coating 4 is disposed on the outer wall of the cutter assembly 2, thereby improving the overall crossability of the cutting balloon and preventing the cutter assembly 2 from injuring the blood vessel.

The connection strength between the hydrophilic coating 4 and the balloon body 1 is insufficient. Therefore, to prevent the hydrophilic coating 4 from falling off, in this embodiment, a coupling agent is disposed between the hydrophilic coating 4 and the outer wall of the balloon body 1. One side of the coupling agent is connected to the outer wall of the balloon body 1 and another side of the coupling agent is connected to the hydrophilic coating 4. The coupling agent is a substance with an amphoteric structure. Some groups in molecules of the coupling agent can react with chemical groups on the surface of the balloon body 1 to form chemical bonds. The other groups in the molecules of the coupling agent are featured by an organophilic nature and can react chemically with organic molecules or generate relatively strong intermolecular interactions with the organic molecules. Thus, the hydrophilic coating 4 and the balloon body 1, which are two fundamentally different materials, are firmly bonded together. In this embodiment, the connection strength between the hydrophilic coating 4 and the balloon body 1 is improved by means of the transition of the coupling agent. In this embodiment, the coupling agent is a silane coupling agent, a titanate coupling agent, or an aluminate coupling agent.

In the related art, the connection strength between a cutter and the balloon is insufficient. When the cutting balloon moves within the blood vessel, a cutting blade has the risk of falling off from the balloon in the body. The falling off of the blade may seriously threaten the life safety of a patient. To solve the preceding problem, the cutting balloon further comprises a first coating 3. The first coating 3 is disposed on the outer wall of the balloon body 1 and is located between the balloon body 1 and the cutter assembly 2. The plurality of cutter assemblies 2 are bonded to the first coating 3. The roughness of the first coating 3 ranges from Ra 0.8 to Ra 15. Specifically, the plurality of cutter assemblies 2 are arranged around the balloon body 1. The first coating 3 is disposed on the outer wall of the balloon body 1 and is located between the balloon body 1 and the cutter assemblies 2. The plurality of cutter assemblies 2 are bonded to the first coating 3 and the roughness of the first coating 3 ranges from Ra 0.8 to Ra 15.

The first coating 3 is disposed on the outer wall of the balloon body 1, and the first coating 3 is capable of modifying the surface of the balloon body 1 to increase the surface roughness. The roughness of the first coating 3 ranges from Ra 0.8 to Ra 15. Thus, the roughness of the outer wall of the balloon body 1 is increased. Therefore, when the cutter assembly 2 is bonded to the first coating 3, the bonding strength between the cutter assembly 2 and the balloon body 1 can be enhanced, the adhesion of the cutter assembly 2 is improved, and the risk that the cutter assembly 2 falls off in the body is reduced. When the cutting balloon moves within the blood vessel, the balloon body 1 is in a deflated state, which facilitates the movement of the cutting balloon. When the cutting balloon moves to the lesion site, the balloon body 1 is inflated, and the cutter assembly 2 cuts a plaque lesion within the blood vessel. The cutting balloon exhibits good crossing performance, the cutter assembly 2 is mounted more firmly, and the tensile strength between the cutter assembly 2 and the balloon body 1 is increased from 5 N to 10 N. Thus, the risk that the cutter assembly 2 falls off in the body is reduced, and the safety factor is higher.

In this embodiment, the first coating 3 is an acidic oxide, specifically including but not limited to a chlorate-sulfuric acid system, a chromic acid-acetic acid system, or an anhydrous chromic acid-tetrachloroethane system. The formation principle of the first coating 3 is described below. The acidic oxide reacts with the outer wall of the balloon body 1 to perform the modification. Molecular chains on the surface of the balloon body 1 are broken, forming dense pits and changing the surface morphology of the substrate. Thus, the roughness is adjusted, and the first coating 3 is formed.

It is to be noted that the forming processes of the first coating 3, the coupling agent, and the hydrophilic coating 4 include one or a combination of dipping, brushing, and spraying, followed by ultraviolet (UV) curing or electron beam curing.

Further, as shown in FIG. 1, the cutter assembly 2 includes a cutter holder 21 and a blade 22, the blade 22 is mounted on one side of the cutter holder 21, and the cutter holder 21 is bonded to the first coating 3. In this embodiment, cutter holders 21 are distributed in 3 to 5 rows in the circumferential direction of the balloon body 1, and multiple blades 22 are disposed on each row of cutter holders 21. The cutter holders 21 may be bonded to the first coating 3 using instant adhesive, light-curable adhesive, or two-component adhesive.

Optionally, the cutter holder 21 extends along the axial direction of the balloon body 1, and two ends of the cutter holder 21 are provided with U-shaped chamfers, thereby reducing blood flow resistance during the advancement of the cutting balloon toward the lesion or during the withdrawal of the cutting balloon and improving the crossability of the cutting balloon.

### Embodiment two

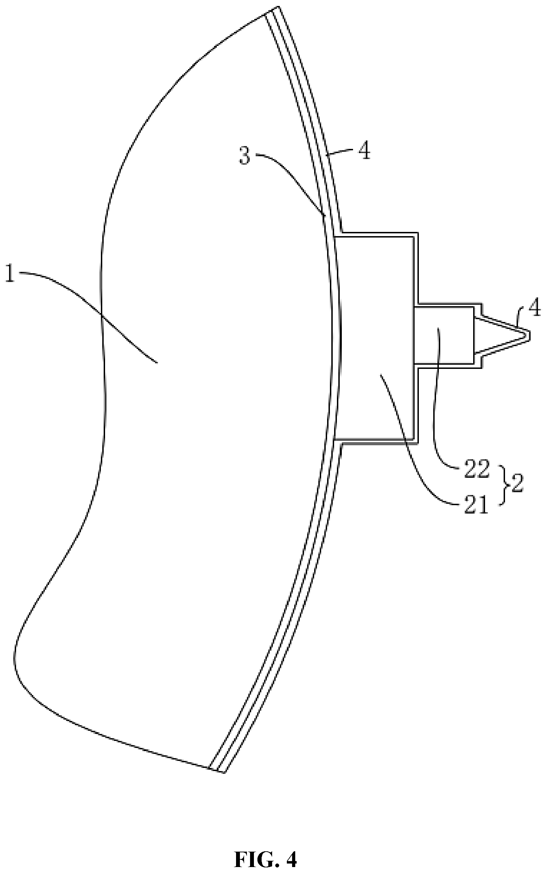

As shown in FIG. 4, this embodiment provides a cutting balloon. The structure of the cutting balloon provided in this embodiment is substantially the same as that in embodiment one, differing only in certain details. Structures that are the same as those in embodiment one are described again in this embodiment.

In this embodiment, the first coating 3 is disposed between the hydrophilic coating 4 and the outer wall of the balloon body 1. The surface roughness of the balloon body 1 is increased so that the contact area between the hydrophilic coating 4 and the surface of the balloon body 1 is increased, thereby improving the connection strength between the hydrophilic coating 4 and the outer wall of the balloon body 1 and preventing the hydrophilic coating 4 from falling off.

Optionally, a coupling agent is disposed between the hydrophilic coating 4 and the first coating 3, and one side of the coupling agent is connected to the first coating 3 and another side of the coupling agent is connected to the hydrophilic coating 4. In this embodiment, the connection strength between the hydrophilic coating 4 and the first coating 3 is improved by means of the transition of the coupling agent.

It is to be noted that the preceding are only preferred embodiments of the present application and the technical principles used therein. It is to be understood by those skilled in the art that the present application is not limited to the embodiments described herein. For those skilled in the art, various apparent modifications, adaptations, and substitutions can be made without departing from the scope of the present application. Therefore, while the present application is described in detail through the preceding embodiments, the present application is not limited to the preceding embodiments and may include more other equivalent embodiments without departing from the concept of the present application. The scope of the present application is determined by the scope of the appended claims.

## Claims

1. A cutting balloon, comprising:
a balloon body (1); and
a plurality of cutter assemblies (2), wherein the plurality of cutter assemblies (2) are arranged on an outer wall of the balloon body (1) and distributed at intervals along a circumferential direction of the balloon body (1);
wherein a hydrophilic coating (4) is disposed on the outer wall of the balloon body (1).

2. The cutting balloon according to claim 1, wherein the hydrophilic coating (4) is disposed on outer walls of the plurality of cutter assemblies (2).

3. The cutting balloon according to claim 1, wherein a coupling agent is disposed between the hydrophilic coating (4) and the outer wall of the balloon body (1), and one side of the coupling agent is connected to the outer wall of the balloon body (1) and another side of the coupling agent is connected to the hydrophilic coating (4).

4. The cutting balloon according to claim 1, further comprising a first coating (3), wherein the first coating (3) is disposed on the outer wall of the balloon body (1).

5. The cutting balloon according to claim 4, wherein the first coating (3) is located between the balloon body (1) and the plurality of cutter assemblies (2), and the plurality of cutter assemblies (2) are bonded to the first coating (3).

6. The cutting balloon according to claim 4, wherein the first coating (3) is disposed between the hydrophilic coating (4) and the outer wall of the balloon body (1).

7. The cutting balloon according to claim 6, wherein a coupling agent is disposed between the hydrophilic coating (4) and the first coating (3), and one side of the coupling agent is connected to the first coating (3) and a side of the coupling agent is connected to the hydrophilic coating (4).

8. The cutting balloon according to claim 4, wherein the first coating (3) is an acidic oxide.

9. The cutting balloon according to claim 3, wherein the coupling agent is a silane coupling agent, a titanate coupling agent, or an aluminate coupling agent.

10. The cutting balloon according to claim 1, wherein a material of the hydrophilic coating (4) is polyacrylamide, polyvinylpyrrolidone, polyethylene glycol, polyvinyl alcohol, or natural polysaccharides.

11. The cutting balloon according to claim 4, wherein the first coating (3) is capable of modifying a surface of the balloon body (1) to increase surface roughness of the balloon body (1).

12. The cutting balloon according to claim 4, wherein roughness of the first coating (3) ranges from Ra 0.8 to Ra 15.

13. The cutting balloon according to claim 5, wherein each cutter assembly (2) among the plurality of cutter assemblies (2) comprises a cutter holder (21) and a blade (22), the blade (22) is mounted on one side of the cutter holder (21), and the cutter holder (21) is bonded to the first coating (3).

14. The cutting balloon according to claim 1, wherein each cutter assembly (2) among the plurality of cutter assemblies (2) comprises a cutter holder (21) and a blade (22), the cutter holder (21) extends along an axial direction of the balloon body (1), and two ends of the cutter holder (21) are provided with U-shaped chamfers.

15. A medical device, comprising the cutting balloon according to any one of claims 1 to 14.
